Europäisches Patentamt

European Patent Office

Office européen des brevets

(19)

(11) Veröffentlichungsnummer: **0 049 345**
**B1**

(12) **EUROPÄISCHE PATENTSCHRIFT**

(45) Veröffentlichungstag der Patentschrift:
**10.12.86**

(21) Anmeldenummer: **81106275.1**

(22) Anmeldetag: **12.08.81**

(51) Int. Cl.⁴: **G 01 N 33/00, G 01 N 37/00**

(54) **Vorrichtung und Anordnung zum Kalibrieren einer Gasmessanlage.**

(30) Priorität: **08.10.80 DE 3038423**

(43) Veröffentlichungstag der Anmeldung:
**14.04.82 Patentblatt 82/15**

(45) Bekanntmachung des Hinweises auf die Patenterteilung:
**10.12.86 Patentblatt 86/50**

(84) Benannte Vertragsstaaten:
**CH FR GB LI SE**

(56) Entgegenhaltungen:
**BE - A - 650 712**
**DE - A - 2 645 736**
**GB - A - 1 174 982**
**US - A - 4 151 738**

(73) Patentinhaber: **AUERGESELLSCHAFT GMBH,**
**Thiemannstrasse 1, D-1000 Berlin 44 (DE)**

(72) Erfinder: **Albrecht, Peter, Hochwaldsteig 10,**
**D-1000 Berlin 22 (DE)**
Erfinder: **Willecke, Raimund, Markgrafenstrasse 29a,**
**D-1000 Berlin 28 (DE)**
Erfinder: **Leins, Rudolf, Grünberger Strasse 2,**
**D-5047 Wesseling (DE)**

## Beschreibung

Die Erfindung betrifft ein Verfahren zum Kalibrieren einer Gasmessanlage, bei dem mindestens ein Messwertgeber nacheinander mit Prüfgasen unterschiedlicher Konzentration beaufschlagt wird, und unter Verwendung der vom Messwertgeber gelieferten, den Prüfgaskonzentrationswerten C entsprechenden Signalen S Fehler bezüglich der Nullpunktlage bzw. der Empfindlichkeit der Anlage korrigiert werden und eine Anordnung zur Durchführung dieses Verfahrens.

Es ist bekannt, dass Gasmessanlagen in gewissen, of sehr kleinen Zeitabständen kalibriert werden müssen. Hierzu werden Prüfgase bekannter Konzentrationen verwendet, mit deren Hilfe der Nullpunkt und die Empfindlichkeit der Anlagen manuell eingestellt werden. Das Kalibrieren erfordert regelmässig mehrere Personen, die die einzelnen Verfahrensabschnitte schrittweise durchführen. Die Kalibrierung ist besonders aufwendig, wenn Messwertgeber und die dazugehörigen Anzeige- oder Registriergeräte anlagenbedingt räumlich weit voneinander getrennt angeordnet sind.

Es ist ein Verfahren zum Kalibrieren von Messanordnungen mit Messwertaufnehmern zur Bestimmung der Konzentration von Gasen bekannt, bei dem der Messwertaufnehmer nacheinander in zwei Umgebungen mit verschiedener, aber bekannter Konzentration des zu bestimmenden Gases gebracht und das Messgerät entsprechend einreguliert wird (DE-A-2 645 736).

Bei einem anderen Verfahren dieser Art (BE-A-650 712), weist die Messanordnung Messwertaufnehmer auf, die mit Versorgungs- und Übertragungsteilen verbunden sind. Die Anordnung ist hierbei mit einem Rechner sowie mit für den Betrieb und die Bedienung der Anordnung erforderlichen Peripheriegeräten versehen.

Es ist weiterhin eine Gasüberwachungseinrichtung zur Überwachung der Konzentration bestimmter Bestandteile eines Gases bekannt (US-A-4 151 738), bei der durch periodisches Beaufschlagen einer Gasprobe bekannter Konzentration auf einen Messwertaufnehmer, die Überwachungseinrichtung automatisch nachgeeicht wird.

Auch ist bei einem anderen automatischen Kalibrierungsverfahren einer Gasmessanlage bekannt (GB-A-1 174 982), einen Messwertgeber in Abständen jeweils mit zwei unterschiedlichen Prüfgasen bekannter Konzentrationen zu beaufschlagen, die als Signale umgesetzt werden und auf einem Messinstrument entsprechende Zeigerausschläge bewirken. Es erfolgt dann die automatische Kalibrierung der Anlage.

Der Erfindung liegt die Aufgabe zugrunde, das bekannte, aufwendige, personalintensive Verfahren zu vereinfachen und eine schnellere Kalibrierung zu ermöglichen.

Diese Aufgabe wird erfindungsgemäss entsprechend der im Anspruch 1 bzw. Anspruch 3 gegebenen Lehre gelöst.

Das erfindungsgemässe Verfahren bietet den Vorteil einer erheblichen Vereinfachung des Kalibriervorganges. Die Kalibrierung kann auch bei Anlagen, deren Messwertgeber weit voneinander installiert sind, von einer einzigen Person durchgeführt werden. Die vom Rechner gespeicherten, jeweils gültigen Kalibrierdaten sind bei Bedarf abruf- bzw. ausdruckbar. Dies kommt sicherheitstechnischen Forderungen entgegen. Der für die Kalibrierung erforderliche Zeitaufwand ist erheblich geringer als bei bekannten Anlagen.

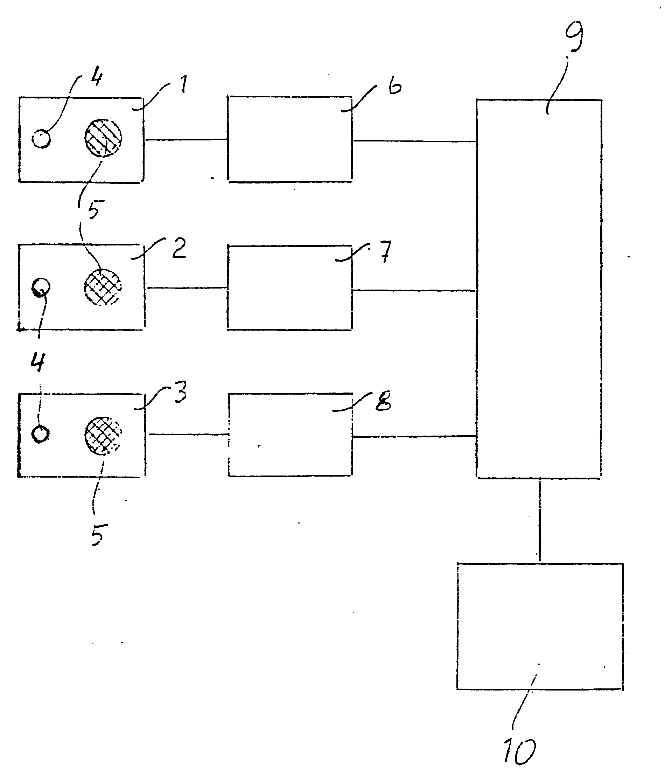

In der beigefügten Zeichnung ist stark vereinfacht das Schema einer erfindungsgemässen Gasmessanlage in Form eines Blockschaubildes dargestellt. Mit 1, 2 und 3 sind Messwertgeber bezeichnet, die mit jeweils einer Taste 4 und einer Gaseintrittsöffnung 5 versehen sind. Die Messwertgeber 1, 2 und 3 stehen mit Versorgungs- und Übertragungsteilen 6, 7 und 8 in Verbindung, die ihrerseits mit den Eingängen eines Rechners 9 verbunden sind.

Beim normalen Betrieb der Anlage werden von den Messwertgebern 1, 2 und 3 Signale $S_n$ an den Rechner geliefert, wobei der Rechner die Signale $S_n$ zyklisch abfragt. Im Rechner 9 wird das von jeweils einem Messwertgeber gelieferte Signal mit mindestens einem vorgegebenen Grenzwert verglichen und bei Überschreiten des jeweiligen Grenzwertes erfolgt eine Alarmmeldung in Form eines Licht- oder Schallsignals und der Ausdruckung eines Alarmprotokolls in einem Drucker 10.

Im Lauf der Betriebszeit verändert sich die Nullpunktlage und die Empfindlichkeit der Anlage. Die Veränderungen werden durch wiederholte Kalibrierungen ausgeglichen.

Zum Kalibrieren werden die einzelnen Messwertgeber 1, 2 und 3 nacheinander mit mindestens zwei Prüfgasen unterschiedlicher Konzentration $C_1$ und $C_2$ beaufschlagt, nachdem zuvor die Prüfgaskonzentrationswerte $C_1$ und $C_2$ in den Rechner eingegeben worden sind. Der jeweilige Messwertgeber liefert nach betätigen seiner Taste 4 den Prüfgaskonzentrationen $C_1$ und $C_2$ entsprechende Signale $S_1$ und $S_2$ an den Rechner 9.

Mit Hilfe der Wertepaare $C_1/S_1$ und $C_2/S_2$ werden die Grenzwerte für die Alarmstufen im Rechner den veränderten Empfindlichkeits- und Nullpunktwerten angepasst.

## Patentansprüche

1. Verfahren zum Kalibrieren einer Gasmessanlage, bei dem mindestens ein Messwertgeber (1, 2, 3) nacheinander mit Prüfgasen unterschiedlicher Konzentration beaufschlagt wird, und unter Verwendung der vom Messwertgeber gelieferten, den Prüfkonzentrationswerten C entsprechenden Signalen S Fehler bezüglich der Nullpunktlage bzw. der Empfindlichkeit der Anlage korrigiert werden, wobei folgende Verfahrensschritte ausgeführt werden:

a) in einen mit einem Kalibrierungsprogramm ausgestatteten Rechner (9) werden mindestens zwei für die Kalibrierung verwendete Prüfgaskonzentrationswerte $C_1$ und $C_2$ eingespeichert;

b) durch Betätigen einer Taste (4) am Messwertgeber werden dem Messwertgeber zugeordnete Alarmstufen im Rechner verriegelt;

c) der Messwertgeber wird mit einem Prüfgas einer ersten vorgegebenen Konzentration $C_1$ beaufschlagt;

d) das vom mit einem Prüfgas der ersten Konzentration $C_1$ beaufschlagten Messwertgeber an den Rechner gelieferte Signal $S_1$ wird von diesem nach einer vorgegebenen Zeitspanne $t_1$ übernommen und gespeichert;

e) der Messwertgeber wird mit einem Prüfgas einer zweiten vorgegebenen Konzentration $C_2$ beaufschlagt;

f) das vom mit dem Prüfgas der zweiten Konzentration $C_2$ beaufschlagten Messwertgeber an den Rechner gelieferte Signal $S_2$ wird von diesem nach einer vorgegebenen Zeitspanne $t_1$ übernommen und gespeichert;

g) die Alarmstufen im Rechner werden nach einer Zeitspanne $t_2$ entriegelt;

h) die vom Messwertgeber danach gelieferten Signale $S_n$ werden im Rechner den aus den Wertepaaren $C_1/S_1$ bzw. $C_2/S_2$ ermittelten Kalibrierwerten entsprechend korrigiert.

2. Verfahren nach Anspruch 1, dadurch gekennzeichnet, dass nach dem Verfahrensschritt d eine Entriegelung der Alarmstufen erfolgt und vor dem Verfahrensschritt e die Taste am Messwertgeber erneut betätigt wird.

3. Anordnung zur Durchführung des Verfahrens nach Anspruch 1 oder 2, mit mindestens einem Messwertgeber (1, 2, 3), der mit einem dazugehörigen Versorgungs- bzw. Übertragungsteil (6, 7, 8) verbunden ist und mit einem Rechner (9) sowie mit für den Betrieb und die Bedienung der Anordnung erforderlichen Peripheriegeräten (10) versehen ist, dadurch gekennzeichnet, dass der Messwertgeber (1, 2, 3) eine Taste (4) aufweist, die einem Schalter zugeordnet ist, der in einem Zweig oder einem Teil eines Zweiges einer zum Messsystem der Gasmessanlage gehörenden Messbrücke angeordnet ist, dass der Rechner (9) dem Messwertgeber zugeordnete Alarmstufen besitzt, die durch Betätigen der Taste (4) am Messwertgeber (1, 2, 3) verriegelt und nach einer Zeitspanne $t_2$ entriegelt werden, und dass der Rechner (9) so ausgebildet ist, dass er die vom Messwertgeber an ihn gelieferten Signale ($S_1$, $S_2$) nach einer vorgegebenen Zeitspanne $t_1$ übernimmt und speichert und dass er die vom Messwertgeber (1, 2, 3) danach gelieferten Signale ($S_n$) den aus den gespeicherten Signalen ermittelten Kalibrierwerten entsprechend korrigiert.

**Claims**

1. Method of calibrating a gas metering system, in which at least one mensuration value transmitter (1, 2, 3) is acted upon successively with test gases of different concentration, and defects in respect of the zero setting position or of the sensitivity of the system are corrected under utilisation of signals S corresponding to the test gas concentration C and supplied by the mensuration value transmitter, wherein the following steps are carried out:

a) at least two test gas concentration values $C_1$ and $C_2$ utilised for calibration are stored in a computer (9) provided with a calibration program;

b) warning stages allocated to the mensuration value transmitter are locked in the computer by operating a key (4) on the mensuration value transmitter;

c) the mensuration value transmitter is acted upon by a test gas at a first preset concentration $C_1$;

d) the signal $S_1$ fed to the computer by the mensuration value transmitter acted upon by a test gas having the first concentration $C_1$, is picked up and stored by the latter after a preset period $t_1$;

e) the mensuration value transmitter is acted upon by a test gas at a second preset concentration $C_2$;

f) the signal $S_2$ fed to the computer by the mensuration value transmitter acted upon by the test gas having the second concentration $C_2$ is picked up and stored by the latter after a preset period $t_1$;

g) the warning stages in the computer are unblocked after a period $t_2$;

h) the signals $S_n$ subsequently supplied by the mensuration value transmitter are corrected in the computer in accordance with the calibration values determined from the pairs of values $C_1/S_1$ and $C_2/S_2$, respectively.

2. Method according to claim 1, characterised in that the release of the warning stages occurs after step d and that the key on the mensuration value transmitter is operated again prior to step e.

3. System for carrying out the method according to claim 1 or 2, comprising at least one mensuration value transmitter (1, 2, 3) which is coupled to an associated supply or transmission section (6, 7, 8) and is provided with a computer (9) as well as with peripherals (10) required for working and operating the system, characterised in that the mensuration value transmitter (1, 2, 3) has a key (4) allocated to a contactor which is situated in a branch or part of a branch of a measuring bridge appertaining to the mensuration system of the gas metering system, that the computer (9) has warning stages allocated to the mensuration value transmitter which – by actuation of the key (4) on the mensuration value transmitter (1, 2, 3) – are locked and released again after a period $t_2$, and that the computer (9) is so arranged that it picks up and stores the signals ($S_1$, $S_2$) supplied to it by the mensuration value transmitter after a preset period $t_1$, and that it corrects the signals ($S_n$) thereafter supplied by the mensuration value transmitter (1, 2, 3) in accordance with the calibration values determined from the signals stored.

**Revendications**

1. Procédé pour calibrer une installation de mesure de gaz, dans lequel au moins un capteur de mesure (1, 2, 3) est successivement soumis à des gaz de contrôle de différentes concentrations, et dans lequel des erreurs relatives à la position zéro ou à la sensibilité de l'installation sont corrigées avec utilisation de signaux S fournis par le capteur et correspondant aux valeurs de concentration C des gaz de contrôle, procédé dans lequel on exécute les opérations suivantes:

a) mémorisation d'au moins deux valeurs de concentration de gaz de contrôle $C_1$ et $C_2$ utilisées à des fins de calibrage dans un calculateur (9) doté d'un programme de calibrage;

b) verrouillage, dans le calculateur, de degrés d'alarme coordonnés au capteur par l'actionnement d'une touche (4) sur le capteur;

c) application au capteur d'un gaz de contrôle d'une première concentration $C_1$ préfixée;

d) reprise, après un laps de temps $t_1$ préfixé, et mémorisation par le calculateur du signal $S_1$ fourni par le capteur, soumis à un gaz de contrôle de la première concentration $C_1$;

e) application au capteur d'un gaz de contrôle d'une deuxième concentration $C_2$ préfixée;

f) reprise, après un laps de temps $t_1$ préfixé, et mémorisation par le calculateur du signal $S_2$ fourni par le capteur, soumis au gaz de contrôle de la deuxième concentration $C_2$;

g) déverrouillage des degrés d'alarme dans le calculateur après un laps de temps $t_2$;

h) correction, dans le calculateur, des signaux $S_n$ fournis ensuite par le capteur, d'après les valeurs de calibrage déterminées à partir des paires de valeurs $C_1/S_2$ respectivement $C_2/S_2$.

2. Procédé selon la revendication 1, caractérisé en ce qu'il comprend un déverrouillage des degrés d'alarme après l'opération d, de même qu'un nouvel actionnement de la touche sur le capteur avant l'opération e.

3. Dispositif pour la mise en œuvre du procédé selon la revendication 1 ou 2, comprenant au moins un capteur de mesure (1, 2, 3) relié à un équipement d'alimentation et de transmission (6, 7, 8) correspondant, un calculateur (9), ainsi que des appareils périphériques (10) nécessaires au fonctionnement et à la commande du dispositif, caractérisé en ce que le capteur (1, 2, 3) présente une touche (4) à laquelle est coordonné un interrupteur disposé dans une branche ou une partie d'une branche d'un pont de mesure appartenant au système de mesure de l'installation de mesure de gaz, que le calculateur (9) possède des degrés d'alarme, coordonnés au capteur, qui sont verrouillés par l'actionnement de la touche (4) sur le capteur (1, 2, 3) et sont déverrouillés après un laps de temps $t_2$, et que le calculateur (9) est conçu de manière qu'il reprenne, après un laps de temps $t_1$ préfixé, et mémorise les signaux ($S_1$, $S_2$) qui lui sont fournis par le capteur et qu'il corrige les signaux ($S_n$) fournis ensuite par le capteur (1, 2, 3) d'après les valeurs de calibrage déterminées à partir des signaux mémorisés.